(19) Europäisches Patentamt
European Patent Office
Office européen des brevets

(11) **EP 3 045 165 B1**

(12) **EUROPEAN PATENT SPECIFICATION**

(45) Date of publication and mention
of the grant of the patent:
**23.09.2020 Bulletin 2020/39**

(51) Int Cl.:
*A61K 9/107* (2006.01)          *A61K 31/4184* (2006.01)
*A61K 31/7068* (2006.01)          *A61P 35/00* (2006.01)

(21) Application number: **16159341.3**

(22) Date of filing: **27.04.2010**

(54) **SELF MICRO-EMULSIFYING ORAL PHARMACEUTICAL COMPOSITION OF HYDROPHILIC DRUG AND PREPARATION METHOD THEREOF**

SELBST-MIKROEMULGIERENDE ORALE PHARMAZEUTISCHE ZUSAMMENSETZUNG AUS EINEM HYDROPHILEN WIRKSTOFF UND ZUBEREITUNGSVERFAHREN DAFÜR

COMPOSITION PHARMACEUTIQUE ORALE AUTO-MICRO-ÉMULSIFIANTE DE MÉDICAMENT HYDROPHILE ET SON PROCÉDÉ DE PRÉPARATION

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **27.04.2009 US 172901 P**

(43) Date of publication of application:
**20.07.2016 Bulletin 2016/29**

(62) Document number(s) of the earlier application(s) in accordance with Art. 76 EPC:
**10769214.7 / 2 425 818**

(73) Proprietor: **Innopharmax, Inc.**
**Taipei City, Taiwan 11492 (TW)**

(72) Inventors:
• **HSU, Chang-Shan**
**11492 / CN (TW)**
• **HAO, Wei-Hua**
**11492 / CN (TW)**

• **WANG, Jong-Jing**
**11492 / CN (TW)**
• **LIN, Tsung-Hsin**
**11492 / CN (TW)**

(74) Representative: **Straus, Alexander et al**
**2K Patent- und Rechtsanwälte - München**
**Keltenring 9**
**82041 Oberhaching (DE)**

(56) References cited:
**WO-A2-2007/103294          CN-A- 1 593 405**
**US-A- 5 707 648          US-A1- 2003 077 297**
**US-A1- 2003 104 048          US-A1- 2004 115 287**

• STRICKLEY ET AL: "Solubilizing expients in oral and injectable formulations", PHARMACEUTICAL RESEARCH, SPRINGER NEW YORK LLC, US, vol. 21, no. 2, 1 February 2004 (2004-02-01), pages 201-230, XP008136709, ISSN: 0724-8741, DOI: 10.1023/B:PHAM.0000016235.32639.23

**Description**

FIELD OF THE INVENTION

The preset invention relates to an oral self micro-emulsifying pharmaceutical composition of hydrophilic drugs.

BACKGROUND OF THE INVENTION

Oral administration is a convenient and user-friendly mode of drug administration, either in the form of a solid or a liquid suspension, which continues to dominate the area of drug delivery technologies. Even though many types of drugs could be administered orally with acceptable efficacy, there remains some problems for some classes of drugs, especially those which are known to have good solubility, but are extensively metabolized in the liver, easily pumped out by the intestinal epithelium (poor permeability) or irritative to the gastric mucosa, such as Class III drugs of Biopharmaceutics Classification System (BCS) provided by the U.S. Food and Drug Administration. Thus, for these drugs, injection administration become the major option to achieve acceptable drug absorption and bioavailability which however leads to increased risk and expenses and further is painful for patients.

A new technique called "self-emulsifying/microemulsifying drug delivery system (SEDDS/SMEDDS)" has been developed in the art which provides a good vehicle to improve bioavailability of hydrophobic drugs and make their oral delivery possible. Normally, the SEDDS/SMEDDS is composed of oil, a surfactant, a cosurfactant or solubilizer, and a hydrophobic drug. The underlying principle of said system is that when the SEDDS/SMEDDS contacts water, it spontaneously forms oil-in-water microemulsions under mild mechanical agitation. Consequently, a drug can be formulated so as to dissolve in a liquid-based formulation that does not contain an aqueous phase. It can then be filled into soft/hard capsules to form solid oral formulations. After being oral administered and contacting gastrointestinal fluids, said formulation is capable of self-emulsifying into microemulsions immediately so as to facilitate the dispersion, dissolution, stability and absorption of the drug, thus improving the bioavailability of said drug. However, for hydrophilic drugs, there are limitations to make them suitable in the SEDDS/SMEDDS. Other known strategies, e.g. liposomes, microparticles or prodrugs, have been reported to enhance the bioavailability of hydrophilic drugs, as described in, for example, U.S. Patent Nos. 7,220,428, 7,053,076, 7,217,735, and 7,309,696, and PCT Publication Nos. WO2004/017944 and WO2007/089043. US2003104048 discloses pharmaceutical dosage forms having a highly hydrophilic fill material and a shell encapsulating the fill material are disclosed and described. Generally, the shell has at least one plasticizing agent therein in order to provide the shell with an effective plasticity. US2004115287 diascloses compositions and methods for providing hydrophobic active agents in a bioavailable form, including cyclosporine. In one aspect, a cyclosporine composition may be formulated that produces an aqueous dispersion containing cyclosporine in both dissolved and undissolved forms.

Therefore, there is still a need to develop an oral dosage form of hydrophilic drugs, especially an oral self-emulsifying pharmaceutical composition with good bioavailability and stability.

BRIEF SUMMARY OF THE INVENTION

In one aspect, the present invention provides an oral self micro-emulsifying pharmaceutical composition that may be a formulation comprising gemcitabine or its pharmaceutically acceptable salt in an amount of about 2.00% (w/w), water in an amount of about 20.00% (w/w); glycerol and PEG together in an amount of about 32.30% (w/w); and polysorbate and oleoyl polyoxylglycerides together in an amount of about 45.70% (w/w), wherein the % (w/w) values are based on the total weight of the pharmaceutical composition; or a formulation comprising gemcitabine in an amount of about 2.00% (w/w); water in an amount of about 20.00% (w/w); propylene glycol and PEG in an amount of about 32.30% (w/w); and polysorbate and oleoyl polyoxylglycerides in an amount of about 45.70% (w/w), wherein the % (w/w) values are based on the total weight of the pharmaceutical composition; or a formulation comprising gemcitabine in an amount of about 1.98% (w/w); water in an amount of about 19.8% (w/w); glycerol and PEG together in an amount of about 31.98% (w/w); polysorbate and oleoyl polyoxylglycerides together in an amount of about 45.25% (w/w); and D-α-tocopheryl polyethylene glycol 1000 succinate (TPGS) in an amount of about 0.99% (w/w), wherein the % (w/w) values are based on the total weight of the pharmaceutical composition.

In another aspect, the invention provides an oral self micro-emulsifying pharmaceutical composition may be a formulation comprising gemcitabine HCl in an amount of 2.00% (w/w), water in an amount of 20.00% (w/w); glycerol in an amount of 2.10 % (w/w); PEG 400 in an amount of 30.20% (w/w); Tween 80 in an amount of 32.30% (w/w) and Labrafil in an amount of 13.40% (w/w), wherein the % (w/w) values are based on the total weight of the pharmaceutical composition; or

a formulation comprising gemcitabine HCl in an amount of 2.00% (w/w); water in an amount of 20.00% (w/w); propylene glycol in an amount of 2.10% (w/w) and PEG 400 in an amount of 30.20% (w/w); Tween 80 in an amount of 32.30% and Labrafil in an amount of 13.40% (w/w), wherein the % (w/w) values are based on the total weight of the pharmaceutical composition; or a formulation comprising gemcitabine HCl in an amount of 1.98% (w/w); water in an amount of 19.8% (w/w); glycerol in an amount of 2.08% (w/w); PEG 400 in an amount of 29.90% (w/w); D-$\alpha$-tocopheryl polyethylene glycol 1000 succinate (TPGS) in an amount of 0.99% (w/w); Tween 80 in an amount of 31.94%; and Labrafil in an amount of 13.31% (w/w), wherein the % (w/w) values are based on the total weight of the pharmaceutical composition; or a formulation comprising gemcitabine HCl in an amount of 2.00% (w/w), water in an amount of 18.03% (w/w); 4.0 N NaOH in an amount of 1.97% (w/w); glycerol in an amount of 2.10 % (w/w); PEG 400 in an amount of 30.20% (w/w); Tween 80 in an amount of 32.30% (w/w) and Labrafil in an amount of 13.40% (w/w), wherein the % (w/w) values are based on the total weight of the pharmaceutical composition; or

a formulation comprising gemcitabine HCl in an amount of 1.98% (w/w); water in an amount of 17.85% (w/w); 4.0 N NaOH in an amount of 1.95% (w/w); propylene glycerol in an amount of 2.08% (w/w); PEG 400 in an amount of 29.90% (w/w); D-$\alpha$-tocopheryl polyethylene glycol 1000 succinate (TPGS) in an amount of 0.99% (w/w); Tween 80 in an amount of 31.94%; and Labrafil in an amount of 13.31% (w/w), wherein the % (w/w) values are based on the total weight of the pharmaceutical composition; or

a formulation comprising gemcitabine HCl in an amount of 2.00% (w/w), water in an amount of 18.26% (w/w); 4.0 N NaOH in an amount of 1.74% (w/w); glycerol in an amount of 2.10 % (w/w); PEG 400 in an amount of 30.20% (w/w); Tween 80 in an amount of 32.30% (w/w) and Labrafil in an amount of 13.40% (w/w), wherein the % (w/w) values are based on the total weight of the pharmaceutical composition; or

a formulation comprising gemcitabine HCl in an amount of 2.00% (w/w), water in an amount of 14.40% (w/w); 4.0 N NaOH in an amount of 5.60% (w/w); glycerol in an amount of 2.10 % (w/w); PEG 400 in an amount of 30.20% (w/w); Tween 80 in an amount of 32.30% (w/w) and Labrafil in an amount of 13.40% (w/w), wherein the % (w/w) values are based on the total weight of the pharmaceutical composition; or

a formulation comprising gemcitabine HCl in an amount of 2.00% (w/w), water in an amount of 14.30% (w/w); 4.0 N NaOH in an amount of 5.70% (w/w); glycerol in an amount of 2.10 % (w/w); PEG 400 in an amount of 30.20% (w/w); Tween 80 in an amount of 32.30% (w/w) and Labrafil in an amount of 13.40% (w/w), wherein the % (w/w) values are based on the total weight of the pharmaceutical composition.

The various embodiments of the present invention are described in details below. Other characteristics of the present invention will be clearly presented by the following detailed description about the various embodiments and claims.

It is believed that a person of ordinary knowledge in the art where the present invention belongs can utilize the present invention to its broadest scope based on the description herein with no need of further illustration. Therefore, the following description should be understood as of demonstrative purpose instead of limitative in any way to the scope of the present invention.

BRIEF DESCRIPTION OF THE SEVERAL VIEWS OF THE DRAWINGS

For the purpose of illustrating the invention, there are shown in the drawings embodiments which are presently preferred. It should be understood, however, that the invention is not limited to the preferred embodiments shown.

In the drawings:

Figure 1 shows the profiles of plasma concentrations of gemcitabine after intravenous and oral administration.
Figure 2 shows the profiles of plasma concentrations of 2',2'-difluorodeoxyuridine (dFdU) after intravenous and oral administration.

DETAILED DESCRIPTION OF THE INVENTION

Unless otherwise defined herein, scientific and technical terms used in connection with the present invention shall have the meanings that are commonly understood by those of ordinary skill in the art. As used herein, the following terms have the meanings ascribed to them unless specified otherwise.

Unless otherwise required by context, singular terms shall include the plural and plural terms shall include the singular. The articles "a" and "an" are used herein to refer to one or more than one (*i.e.*, at least one) of the grammatical object of the article. By way of example, "an element" means one element or more than one element.

Disclosed is an oral self micro-emulsifying pharmaceutical composition of a hydrophilic drug which, in addition to the hydrophilic drug, one or more solvents for solving the hydrophilic drug to form a drug-solvent solution, and a surfactant system comprising one or more surfactants exhibiting a HLB value ranging from about 8 to about 17, comprises one or more hydrophilic carrier which are compatible with said drug-solvent solution and said surfactant system. The oral self-emulsifying pharmaceutical composition according to the invention exhibits excellent bioavailability of the drug through oral administration which is comparable to that of the drug through intravenous injection.

As used herein, the term "self micro-emulsifying" is to describe a formulation which when contacting an aqueous medium (such as mixed with water) produces a fine oil-water emulsion. The self micro-emulsifying pharmaceutical composition of the invention, when contacting an aqueous medium, may form an emulsion with a mean particle size of less than 800 nm, less than 400 nm, less than 200 nm, or less than 100 nm. The self micro-emulsifying pharmaceutical composition of the invention, when contacting an aqueous medium, may form an emulsion with a mean particle size of about 10 nm.

As used herein, the term "therapeutically effective amount" means a dose of the drug as used that is effective in exerting a therapeutic effect, particularly a dose of the drug which, after absorption into the body through the walls of gastrointestinal (GI) tract, yields a drug concentration in the blood effective in exerting a therapeutic effect on a target organ. Persons skilled in the art will understand that the amounts of the drug presented in the composition vary with the particular situation, including but not limited to, the species and dosage form of the drug and the size, age and condition of the subject.

The term "pharmaceutically acceptable salt" as used herein includes, but is not limited to, acid addition salts that substantially retain the biological effectiveness and properties of the free bases. Such acid addition salts may be formed with inorganic acids such as hydrochloric acid, hydrobromic acid, sulfuric acid, nitric acid, phosphoric acid and the like, and organic acids such as acetic acid, propionic acid, pyruvic acid, maleic acid, malonic acid, succinic acid, fumaric acid, tartaric acid, citric acid, benzoic acid, mandelic acid, methanesulfonic acid, ethanesulfonic acid, *p*-toluenesulfonic acid, salicylic acid, trifluoroacetic acid and the like.

The solvent(s) as used herein includes but is not limited to water, ethanol, polyethylene glycol (PEG), isopropanol (IPA), 1,2-propanediol (propylene glycol), glycerol, and acetic acidIn one embodiment, the self micro-emulsifying pharmaceutical composition of the invention contains water as a solvent.

The surfactant(s) to be used herein may be cationic surfactants, anionic surfactants, or nonionic surfactants. Examples of the surfactant(s) include but are not limited to polysorbate, poloxamers, oleoyl polyoxylglycerides (such as Labrafil M1944CS), linoleoyl polyoxylglycerides (such as Labrafil M2125CS), caprylocaproyl polyoxylglycerides (such as Labrasol), polyoxyethylene castor oil derivatives (such as PEG 40 hydrogenated castor oil, Cremophor EL or Cremophor RH), polyoxyethylene alkyl ethers (such as Brij), sorbitan fatty acid esters (such as Spans), glyceryl monooleate (such as PECEOL®), glyceryl monolinoleate (such as Maisine® 35-1), medium-chain triglycerides (MCT), polyglyceryl oleate (such as Plurol® Oleique CC497), lauroyl polyoxylglyceride (such as Gelucire® 44/14), stearoyl polyoxylglycerides (such as Gelucire® 50/13), propylene glycol dicaprylocaprate (such as Labrafac® PG), propylene glycol laurate (such as Lauroglycol® FCC), propylene glycol monolaurate (such as Lauroglycol® 90), propylene glycol caprylate (such as Capryol PGMC) and propylene glycol monocaprylate (such as Capryol 90).

As used herein, the term "compatible" means that the one or more hydrophilic carriers are mixable or dispersed with the above-mentioned drug-solvent solution and the surfactant system so as to form a stable homogenous solution. Particularly, each of the one or more hydrophilic carriers as used herein is capable of dissolving about 1 part of a given hydrophilic drug or its pharmaceutically acceptable salt in about 10 to about 10,000 parts of the hydrophilic carrier. More particularly, the one or more hydrophilic carriers as used herein may be selected from the group consisting of (a) a first hydrophilic carrier capable of dissolving about 1 part of a given hydrophilic drug or its pharmaceutically acceptable salt in about 10 to about 30 parts of the first hydrophilic carriers (soluble); (b) a second hydrophilic carrier capable of dissolving about 1 part of a given hydrophilic drug or its pharmaceutically acceptable salt in about 30 to about 100 parts of the second hydrophilic carrier (sparingly soluble); (c) a third hydrophilic carrier capable of dissolving about 1 part of a given hydrophilic drug or its pharmaceutically acceptable salt in about 100 to about 1,000 parts of the third hydrophilic carrier (slightly soluble); (d) a forth hydrophilic carrier capable of dissolving about 1 part of a given hydrophilic drug or its pharmaceutically acceptable salt in about 1,000 to about 10,000 parts of the fourth hydrophilic carrier (very slightly soluble); and (e) any combination thereof. Examples of the hydrophilic carrier(s) as used herein include but are not limited to polysorbate, ethanol, polyethylene glycol (PEG) such as PEG200, PEG300, PEG400, PEG600, PEG 1000, PEG2000, PEG3000, PEG4000, PEG6000, or PEG8000, glycerol, 1,2-propanediol (propylene glycol), propylene carbonate (PC), and diethylene glycol monoethyl ether (such as Transcutol® HP).

Further, in some instances, it may be particularly advantageous to use certain combinations of the solvent(s) and the hydrophilic carrier(s), for examplea third or fourth hydrophilic carrier, a second solvent in combination with third hydrophilic carrier, a third solvent in combination with a second or third hydrophilic carrier, or a fourth solvent in combination with a third hydrophilic carrier. In addition, the solvent(s) and the hydrophilic carrier(s) are particularly together present in an amount ranging from about 25% to about 65% (w/w), more particularly about 40% to about 60% (w/w), and even more particularly about 50% (w/w), based on the weight of the pharmaceutical composition of the invention. Specifically, the solvent(s) and the hydrophilic carrier(s) are present at the ratio of about 1: 0.1 to about 1: 9 by weight in the pharmaceutical composition of the invention. More specifically, if the pharmaceutical composition of the invention is in the form of oral solution, the solvent(s) and the hydrophilic carrier(s) are present at the ratio of about 1: 0.1 to about 1: 2 by weight in the pharmaceutical composition of the invention; and if the pharmaceutical composition of the invention is in the form of capsule, the solvent(s) and the hydrophilic carrier(s) are present at the ratio of about 1: 1 to about 1: 9 by weight in the pharmaceutical composition of the invention. On the other hand, the hydrophilic carrier(s) and the surfactant system are particularly together present in an amount ranging from about 50% to about 95% (w/w), more particularly about 65% to about 85% (w/w), and even more particularly about 75% (w/w), based on the weight of the pharmaceutical composition of the invention. Specifically, the hydrophilic carrier(s) and the surfactant system are present at the ratio of about 1: 0.3 to about 1: 32.5, more specifically about 1:1 to about 1:20, and even more specifically about 1: 1.5 by weight in the pharmaceutical composition of the invention.

The solvent(s), the hydrophilic carrier(s) and the surfactant system may be present at the ratio of about 2: 3: 4.5 by weight in the pharmaceutical composition of the invention.

In addition, the self micro-emulsifying pharmaceutical composition of the invention may optionally include other components such as an antioxidant e.g. D-α-tocopheryl polyethylene glycol 1000 succinate (TPGS).

In a certain embodiment, the self micro-emulsifying pharmaceutical composition of the invention comprises gemcitabine or its pharmaceutically acceptable salt, water, glycerol, PEG, polysorbate, and oleoyl polyoxylglycerides. In a specific example, gemcitabine is present in an amount of about 2.00% (w/w) based on the weight of the pharmaceutical composition; water is present in an amount of about 20.00% (w/w) based on the weight of the pharmaceutical composition; glycerol and PEG are together present in an amount of about 32.30% (w/w) based on the weight of the pharmaceutical composition; and polysorbate, and oleoyl polyoxylglycerides are together present in an amount of about 45.70% (w/w) based on the weight of the pharmaceutical composition.

In a certain embodiment, the self-emulsifying pharmaceutical composition of the invention comprises gemcitabine or its pharmaceutically acceptable salt, water, propylene glycol, PEG, polysorbate, and oleoyl polyoxylglycerides. In a specific example, gemcitabine is present in an amount of about 2.00% (w/w) based on the weight of the pharmaceutical composition; water is present in an amount of about 20.00% (w/w) based on the weight of the pharmaceutical composition; propylene glycol and PEG are together present in an amount of about 32.30% (w/w) based on the weight of the pharmaceutical composition; and polysorbate, and oleoyl polyoxylglycerides are together present in an amount of about 45.70% (w/w) based on the weight of the pharmaceutical composition.

In a certain embodiment, the self-emulsifying pharmaceutical composition of the invention comprises gemcitabine or its pharmaceutically acceptable salt, water, glycerol, PEG, polysorbate, oleoyl polyoxylglycerides, and TPGS. In a specific example, gemcitabine is present in an amount of about 1.98% (w/w) based on the weight of the pharmaceutical composition; water is present in an amount of about 19.8% (w/w) based on the weight of the pharmaceutical composition; glycerol and PEG are together present in an amount of about 31.98% (w/w) based on the weight of the pharmaceutical composition; polysorbate and oleoyl polyoxylglycerides are together present in an amount of about 45.25% (w/w) based on the weight of the pharmaceutical composition; and TPGS is present in an amount of about 0.99% (w/w) based on the weight of the pharmaceutical composition.

In addition, the self micro-emulsifying pharmaceutical composition of the invention is optionally adjusted to have a pH above the dissociation constant (pKa) of the hydrophilic drug contained therein to increase stability during storage. The self micro-emulsifying pharmaceutical composition of the invention containing gemcitabine may further be adjusted to have a pH above 4.0, e.g., at pH 4-5, 5-6, 6-7, or 7-8.

The self micro-emulsifying pharmaceutical composition according to the invention exhibits excellent bioavailability of the drug through oral administration which is comparable to that of the drug through intravenous injection. In a specific example, the self-emulsifying pharmaceutical composition according to the invention shows relative bioavailability of about 89% through oral administration as compared to the conventional formulation through injection (see Example 4 below).

Further, the self micro-emulsifying pharmaceutical composition according to the invention also exhibits good stability during storage, which particularly means that there is no substantial phase separation, material precipitation, texture change, or degradation of an active ingredient contained therein during a certain storage period. The term "no substantial degradation of an active ingredient contained therein" means that the amount of the active ingredient lost in the pharmaceutical composition of the invention after being stored for a certain period of time is less than 20%, preferably less than 10%, and more preferably less than about 5 % of the original amount of the active ingredient in the pharmaceutical composition.

The pharmaceutical compositions of this invention may be orally administered in any orally acceptable dosage form including, but not limited to, capsules, tablets, powders or coated granules, which may contain pharmaceutical excipients known in the art such as binders, fillers, filler/binders, adsorbents, moistening agents, disintegrants, lubricants and the like as needed.

In certain embodiments of the invention, the pharmaceutical composition is encapsulated in a sealed soft or hard capsule. The capsule is typically of a kind which is dissolved in a particular region of the GI tract releasing its content there. An example of such a capsule is an enteric-coated soft or hard gelatin capsule. Enteric coating, as known *per se*, is coating with a substance or a combination of substances that resists dissolution in gastric fluid but disintegrates in the intestine.

The pharmaceutical composition of the present invention can be prepared by mixing gemcitabine with the one or more solvents, the hydrophilic carriers, and the surfactant system using any standard method commonly used in the art in view of the present disclosure, in the examples below.

The present invention will now be described more specifically with reference to the following embodiments, which are provided for the purpose of demonstration rather than limitation.

**Example 1: Preparation of self-emulsifying pharmaceutical compositions of the invention**

1. Formulation I

Gemcitabine hydrochloride (100 mg) was added to distilled water (1,000 mg), glycerol (105 mg) and PEG 400 (1,510 mg) and agitated until completely dissolved to form Solution A. Tween 80 (1,613 mg) and Labrafil M1944CS (672mg) were homogenously mixed in another container to form Solution B. Solution A was then poured into Solution B, and agitated until a clear solution was obtained to form Formulation I, which was further made into a hard/soft capsule using a well-known method in the art.

Table 1 shows the composition of Formulation I.

Table 1

| | Component | weight (mg) | percentage (%) |
|---|---|---|---|
| Formulation I pH 1-2 HLB of surfactants (11.76) | gemcitabine HCl | 100 | 2.00 |
| | Water | 1,000 | 20.00 |
| | glycerol | 105 | 2.10 |
| | PEG 400 | 1,510 | 30.20 |
| | Tween 80 | 1,613 | 32.30 |
| | Labrafil M1944 CS | 672 | 13.40 |
| | Total | 5,000 | 100.00 |

2. Formulation II

First, gemcitabine hydrochloride (100 mg) was added to distilled water (1,000 mg), propylene glycol (105 mg) and PEG 400 (1,510 mg) and agitated until completely dissolved to form Solution A. Tween 80 (1,613 mg) and Labrafil M1944CS (672 mg) were homogenously mixed in another container to form Solution B. Solution A was then poured into Solution B and agitated until a clear solution was obtained to form Formulation II which was further made into a hard/soft capsule using a well-known method in the art.

Table 2 shows the composition of Formulation II.

Table 2

| | component | weight (mg) | Percentage (%) |
|---|---|---|---|
| Formulation II pH 1-2 HLB of surfactants (11.76) | gemcitabine HCl | 100 | 2.00 |
| | water | 1,000 | 20.00 |
| | propylene glycol | 105 | 2.10 |
| | PEG 400 | 1,510 | 30.20 |
| | Tween 80 | 1,613 | 32.30 |
| | Labrafil M1944 CS | 672 | 13.40 |
| | Total | 5,000 | 100.00 |

3. Formulation III

Gemcitabine hydrochloride (100 mg) was added to distilled water (1,000 mg), glycerol (105 mg), PEG 400 (1,510 mg) and TPGS (50 mg), and agitated until completely dissolved to form Solution A. Tween 80 (1,613 mg) and Labrafil M1944CS (672 mg) were homogenously mixed in another container to form Solution B. Solution A was then poured into Solution B and agitated until a clear solution was obtained to form Formulation III which was further made into a hard/soft capsule using a well-known method in the art. Table 3 shows the composition of Formulation III.

Table 3

|  | component | weight (mg) | Percentage (%) |
|---|---|---|---|
| Formulation III pH 1-2 HLB of surfactants (11.76) | gemcitabine HCl | 100 | 1.98 |
|  | Water | 1,000 | 19.80 |
|  | glycerol | 105 | 2.08 |
|  | PEG 400 | 1,510 | 29.90 |
|  | TPGS | 50 | 0.99 |
|  | Tween 80 | 1,613 | 31.94 |
|  | Labrafil M1944 CS | 672 | 13.31 |
|  | Total | 5,050 | 100.00 |

### 4. Formulation IV

Gemcitabine hydrochloride (100 mg) was added into distilled water (901.3mg), 4.0 N NaOH solution (98.7 mg) glycerol (105 mg), PEG 400 (1,510 mg), and agitated until completely dissolved to form Solution A. Tween 80 (1,613mg) and Labrafil M1944CS (672mg) were homogenously mixed in another container to form Solution B. Solution A was then poured into Solution B, and agitated until a clear solution was obtained to form Formulation IV which was further made into a hard/soft capsule using a well-known method in the art. Table 4 shows the composition of Formulation IV.

Table 4

|  | component | weight (mg) | Percentage (%) |
|---|---|---|---|
| Formulation IV pH 5-6 HLB of surfactants (11.76) | gemcitabine HCl | 100 | 2.00 |
|  | water | 901.3 | 18.03 |
|  | 4.0 N NaOH | 98.7 | 1.97 |
|  | glycerol | 105 | 2.10 |
|  | PEG 400 | 1,510 | 30.20 |
|  | Tween 80 | 1,613 | 32.30 |
|  | Labrafil M1944 CS | 672 | 13.40 |
|  | Total | 5,000 | 100.00 |

### 5. Formulation V

Gemcitabine hydrochloride (100 mg) was added to distilled water (901.3 mg), 4.0 N NaOH solution (98.7mg), propylene glycol (105 mg), PEG 400 (1,510mg), TPGS (50mg), and agitated until completely dissolved to form Solution A. Tween 80 (1,613mg) and Labrafil M1944CS (672mg) were homogenously mixed in another container to form Solution B. Solution A was then poured into Solution B, and agitated until a clear solution was obtained to form Formulation V which was further made into a hard/soft capsule using a well-know method in the art. Table 5 shows the composition of Formulation V.

Table 5

|  | component | weight (mg) | Percentage (%) |
|---|---|---|---|
| Formulation V pH 5-6 HLB of surfactants (11.76) | gemcitabine HCl | 100 | 1.98 |
|  | water | 901.3 | 17.85 |
|  | 4.0 N NaOH | 98.7 | 1.95 |
|  | propylene Glycol | 105 | 2.08 |
|  | PEG 400 | 1,510 | 29.90 |
|  | TPGS | 50 | 0.99 |
|  | Tween 80 | 1,613 | 31.94 |
|  | Labrafil M1944 CS | 672 | 13.31 |
|  | Total | 5050 | 100 |

### 6. Formulation VI

Gemcitabine hydrochloride (100 mg) was added to distilled water (913.28 mg), 4.0 N NaOH solution (86.72 mg), glycerol (105 mg), and PEG 400 (1,510 mg), and agitated until completely dissolved to form Solution A. Tween 80 (1,613 mg) and Labrafil M1944CS (672 mg) were homogenously mixed in another container to form Solution B. Solution A was then poured into Solution B, and agitated until a clear solution was obtained to form Formulation VI which was further made into a hard/soft capsule using a well-know method in the art. Table 6 shows the composition of Formulation VI.

Table 6

|  | component | weight (mg) | Percentage (%) |
|---|---|---|---|
| Formulation VI pH 4-5 HLB of surfactants (11.76) | gemcitabine HCl | 100 | 2.00 |
|  | water | 913.28 | 18.26 |
|  | 4.0 N NaOH | 86.72 | 1.74 |
|  | glycerol | 105 | 2.10 |
|  | PEG 400 | 1,510 | 30.20 |
|  | Tween 80 | 1,613 | 32.30 |
|  | Labrafil M1944 CS | 672 | 13.40 |
|  | Total | 5000 | 100 |

### 7. Formulation VII

Gemcitabine hydrochloride (100 mg) was added to distilled water (720.21 mg), 4.0 N NaOH solution (279.79 mg), glycerol (105 mg), and PEG 400 (1,510 mg), and agitated until completely dissolved to form Solution A. Tween 80 (1,613 mg) and Labrafil M1944CS (672 mg) were homogenously mixed in another container to form Solution B. Solution A was then poured into Solution B, and agitated until a clear solution was obtained to form Formulation VII which was further made into a hard/soft capsule using a well-know method in the art.

Table 7 shows the composition of Formulation VII.

Table 7

|  | component | weight (mg) | Percentage (%) |
|---|---|---|---|
| Formulation VII pH 6-7 HLB of surfactants (11.76) | gemcitabine HCl | 100 | 2.00 |
|  | water | 720.21 | 14.40 |
|  | 4.0 N NaOH | 279.79 | 5.60 |
|  | glycerol | 105 | 2.10 |
|  | PEG 400 | 1,510 | 30.20 |
|  | Tween 80 | 1,613 | 32.30 |
|  | Labrafil M1944 CS | 672 | 13.40 |
|  | Total | 5000 | 100 |

### 8. Formulation VIII

Gemcitabine hydrochloride (100 mg) was added to distilled water (715 mg), 4.0 N NaOH solution (285 mg), glycerol (105 mg), and PEG 400 (1,510 mg), and agitated until completely dissolved to form Solution A. Tween 80 (1,613 mg) and Labrafil M1944CS (672 mg) were homogenously mixed in another container to form Solution B. Solution A was then poured into Solution B, and agitated until a clear solution was obtained to form Formulation VIII which was further made into a hard/soft capsule using a well-know method in the art.

Table 8 shows the composition of Formulation VIII.

Table 8

| | component | weight (mg) | Percentage (%) |
|---|---|---|---|
| Formulation VIII pH 7-8 HLB of surfactants (11.76) | Gemcitabine HCl | 100 | 2.00 |
| | water | 715 | 14.30 |
| | 4.0 N NaOH | 285 | 5.70 |
| | glycerol | 105 | 2.10 |
| | PEG 400 | 1,510 | 30.20 |
| | Tween 80 | 1,613 | 32.30 |
| | Labrafil M1944 CS | 672 | 13.40 |
| | Total | 5000 | 100.00 |

**Example 2: Measurement of particle size of self-emulsifying pharmaceutical compositions of the invention**

The particle size of the microemulsion droplets of Formulations I to VIII was measured. Briefly, 250 ml distilled water was poured into the dissolution mini vessel and heated to 37°C. Once the temperature reached 37°C, 0.25 ml of the formulation to be tested was added into the vessel. The mixture was agitated by paddle at 100 rpm for 10 minutes. After 10 minutes, transferred about 1 ml mixture to a sample cuvette, then measured the particle size of microemulsion droplets by Zetasizer (Zetasizer Nano-ZS,, Malvern Inst., UK) which following the instructions given in the manuals provided by the manufacturer. Table 9 shows the particle sizes of the microemulsions formed by the pharmaceutical compositions of the present invention as measured.

Table 9

| Droplet Particle Sizes (Z-average: d. nm) | |
|---|---|
| Formulation I | 10.13 |
| Formulation II | 9.57 |
| Formulation III | 12.65 |
| Formulation IV | 13.35 |
| Formulation V | 16.15 |
| Formulation VI | 64.58 |
| Formulation VII | 89.45 |
| Formulation VIII | 83.18 |

**Example 3: Preparation of a comparative formulation for injection**

Gemcitabine hydrochloride (53 mg) was added into a normal saline (4,947 mg), and agitated until completely dissolved to form a comparative formulation (5000 mg). Table 10 shows the composition of the comparative formulation.

Table 10

| | component | weight (mg) | percentage (%) |
|---|---|---|---|
| Comparative formulation (powder, intravenous injection dosage form) | gemcitabine HCl | 53 | 1.06% |
| | water | 4947 | 98.94% |
| | Total | 5,000 | 100.00% |

**Example 4: Bioassay**

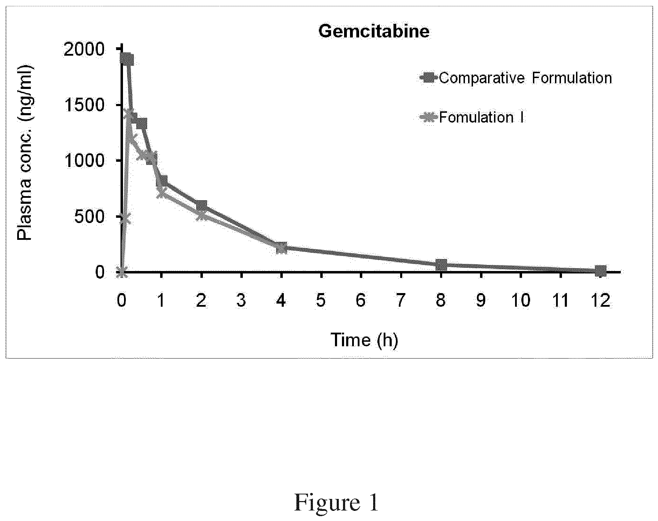

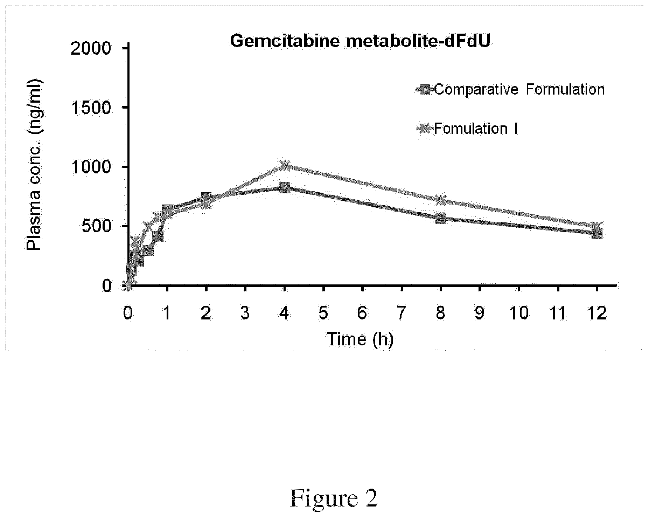

Formulation I (1 mg/kg) as prepared in Example 1 were administrated to a beagle dog via feeding tube; and the comparative formulation (1 mg/kg) as prepared in Example 3 was administrated to another beagle dog by intravenous injection. The blood of the dogs was collected at 5, 10, 15, 30, and 45 minutes, and 1, 2, 4, 8, and 12 hours after the administration, respectively. The collected blood was added into a tube with a reaction terminator and an anticoagulant, and the mixture was subsequently centrifuged to obtain the plasma. Gemcitabine and its main metabolite were analyzed by LC/MS/MS (liquid chromatography/ mass spectrometer). Figures 1 and 2 and Tables 11 and 12 shows the results of the bioassay.

Table 11: Non-compartment model analysis of plasma gemcitabine pharmacokinetic parameters

| Route | i.v. injection | oral administration |
|---|---|---|
| Formulations (1 mg/kg) | Comparative Formulation | Formulation I |
| $AUC_{0-t}$ (mg*h/L) | 3.57 | 2.62 |
| $AUC_{0-\infty}$ (mg*h/L) | 3.60 | 3.22 |
| $C_{max}$ (mg/L) | 1.92 | 1.62 |
| $T_{max}$ (h) | 0.08 | 0.17 |
| $T_{1/2}$ (h) | 1.79 | 1.71 |

Table 12: Non-compartment model analysis of plasma dFdU pharmacokinetic parameters

| Route | i.v. injection | oral administration |
|---|---|---|
| Formulation (1 mg/kg) | Comparative Formulation | Formulation I |
| $AUC_{0-t}$ (mg*h/L) | 7.26 | 9.43 |
| $AUC_{0-\infty}$ (mg*h/L) | 12.81 | 17.08 |
| $C_{max}$ (mg/L) | 0.82 | 1.01 |
| $T_{max}$ (h) | 4.00 | 4.00 |
| $T_{1/2}$ (h) | 8.77 | 9.44 |

The results show that gemcitabine can be well absorbed in the animals through oral administration of the self micro-emulsifying pharmaceutical composition of the invention. The relative bioavailability of the self micro-emulsifying pharmaceutical composition of the invention is about 89% (3.22/3.60) as compared to the comparative formulation through i.v. injection. Also, the plasma profile of dFdU of the self micro-emulsifying pharmaceutical composition of the invention is similar to that of the comparative formation, suggesting less first-pass metabolic effects compared to that of other oral formulations of gemcitabine in the prior art. The present invention for the first time provides a self micro-emulsifying pharmaceutical composition of gemcitabine with comparable bioavailability to that of conventional formulations through *i.v.* injection as used in the art.

**Example 4: Stability test method**

Formulations I to VIII of Example 1 were subjected to a stability test which can be conducted based on a conventional method known in the art. Briefly, about 2 g of the formulation was added into a vial (4 ml) which was then filled with nitrogen and sealed with Teflon septum and aluminum cap. The sealed vials were subsequently put in a Constant Temperature and Humidity Chamber (25 °C 60% RH or 40°C 75%RH) for at least 30 days. On each time point, some of the vials were taken out and the samples inside were poured into a volumetric flask (100 ml). Residual samples were eluted with distilled water and collected in the flask as well. The flask was finally filled with water to 100 ml. HPLC analysis was then conducted to determine the amount (w) of gemcitabine in the samples collected in the flask. The degradation rate (%) of gemcitabine is calculated as below:

$$1 - \frac{\text{Amount of the hydrophilic drug at Day 7, 14, 21 or 30}}{\text{Amount of the hydrophilic drug at Day 0}} \times 100\%$$
.

Table 13 shows the results of the degradation rate of Formulations I to VIII of the invention.

| | | | | Formulations | | | | | |
|---|---|---|---|---|---|---|---|---|---|
| | | I (pH1-2) | II (pH1-2) | III (pH1-2) | IV (pH5-6) | V (pH5-6) | VI (pH4-5) | VII (pH6-7) | VIII (pH7-8) |
| Time | Condition | | | | degradation rate (%) | | | | |
| 7 day | 1 | 2.80% | 3.73% | 3.79% | 4.09% | 3.86% | 6.12% | 4.16% | 4.75% |
| | 2 | 4.64% | 5.39% | 5.67% | 4.60% | 3.62% | 4.11% | 3.73% | 4.97% |

(continued)

| | | I (pH1-2) | II (pH1-2) | III (pH1-2) | IV (pH5-6) | V (pH5-6) | VI (pH4-5) | VII (pH6-7) | VIII (pH7-8) |
|---|---|---|---|---|---|---|---|---|---|
| | | | | | Formulations | | | | |
| Time | Condition | | | | degradation rate (%) | | | | |
| 14 day | 1 | 2.79% | 1.48% | 2.29% | 3.61% | 4.90% | 5.21% | 6.92% | 5.73% |
| | 2 | 6.53% | 7.59% | 5.76% | 4.02% | 2.32% | 4.77% | 6.06% | 6.24% |
| 21 day | 1 | 5.37% | 6.76% | 9.83% | 4.35% | 7.95% | ND | ND | ND |
| | 2 | 14.80% | 10.21% | 12.71% | 6.25% | 8.76% | ND | ND | ND |
| 30 day | 1 | 5.80% | 5.82% | 6.31% | 4.48% | 4.67% | 5.51% | 5.17% | 4.75% |
| | 2 | 13.61% | 12.39% | 12.60% | 5.19% | 6.75% | 5.96% | 4.26% | 6.56% |

Condition 1 is 25 °C and 60% relative humidity.
Condition 2 is 40°C and 75% relative humidity.
ND means not determined.

According to the results, Formulations I to VIII of the invention exhibit high stability at room temperature (25 °C) for at least 30 days (less than 10% of the degradation rate), and among them Formulations IV to VIII (pH above 4) exhibit high stability at 40 °C for at least 30 days (less than 10% of the degradation rate).

This invention is not limited to the specific embodiments described herein, and the right is reserved to the illustrated embodiments and all modifications coming within the scope of the following claims.

## Claims

1. An oral self micro-emulsifying pharmaceutical composition, which is selected from the group consisting of:

   (i) a formulation comprising gemcitabine or its pharmaceutically acceptable salt in an amount of 2.00% (w/w), water in an amount of 20.00% (w/w); glycerol and PEG together in an amount of 32.30% (w/w); and polysorbate and oleoyl polyoxylglycerides together in an amount of 45.70% (w/w), wherein the % (w/w) values are based on the total weight of the pharmaceutical composition;
   (ii) a formulation comprising gemcitabine in an amount of 2.00% (w/w); water in an amount of 20.00% (w/w); propylene glycol and PEG in an amount of 32.30% (w/w); and polysorbate and oleoyl polyoxylglycerides in an amount of 45.70% (w/w), wherein the % (w/w) values are based on the total weight of the pharmaceutical composition; and
   (iii) a formulation comprising gemcitabine in an amount of 1.98% (w/w); water in an amount of 19.8% (w/w); glycerol and PEG together in an amount of 31.98% (w/w); polysorbate and oleoyl polyoxylglycerides together in an amount of 45.25% (w/w); and D-$\alpha$-tocopheryl polyethylene glycol 1000 succinate (TPGS) in an amount of 0.99% (w/w), wherein the % (w/w) values are based on the total weight of the pharmaceutical composition.

2. An oral self micro-emulsifying pharmaceutical composition, which is selected from the group consisting of:

   (i) a formulation comprising gemcitabine HCl in an amount of 2.00% (w/w), water in an amount of 20.00% (w/w); glycerol in an amount of 2.10 % (w/w); PEG 400 in an amount of 30.20% (w/w); Tween 80 in an amount of 32.30% (w/w) and Labrafil in an amount of 13.40% (w/w), wherein the % (w/w) values are based on the total weight of the pharmaceutical composition;
   (ii) a formulation comprising gemcitabine HCl in an amount of 2.00% (w/w); water in an amount of 20.00% (w/w); propylene glycol in an amount of 2.10% (w/w) and PEG 400 in an amount of 30.20% (w/w); Tween 80 in an amount of 32.30% and Labrafil in an amount of 13.40% (w/w), wherein the % (w/w) values are based on the total weight of the pharmaceutical composition;
   (iii) a formulation comprising gemcitabine HCl in an amount of 1.98% (w/w); water in an amount of 19.8% (w/w); glycerol in an amount of 2.08% (w/w); PEG 400 in an amount of 29.90% (w/w); D-$\alpha$-tocopheryl polyethylene glycol 1000 succinate (TPGS) in an amount of 0.99% (w/w); Tween 80 in an amount of 31.94%; and Labrafil in an amount of 13.31% (w/w), wherein the % (w/w) values are based on the total weight of the pharmaceutical composition;
   (iv) a formulation comprising gemcitabine HCl in an amount of 2.00% (w/w), water in an amount of 18.03% (w/w); 4.0 N NaOH in an amount of 1.97% (w/w); glycerol in an amount of 2.10 % (w/w); PEG 400 in an amount of 30.20% (w/w); Tween 80 in an amount of 32.30% (w/w) and Labrafil in an amount of 13.40% (w/w), wherein the % (w/w) values are based on the total weight of the pharmaceutical composition;

(v) a formulation comprising gemcitabine HCl in an amount of 1.98% (w/w); water in an amount of 17.85% (w/w); 4.0 N NaOH in an amount of 1.95% (w/w); propylene glycerol in an amount of 2.08% (w/w); PEG 400 in an amount of 29.90% (w/w); D-α-tocopheryl polyethylene glycol 1000 succinate (TPGS) in an amount of 0.99% (w/w); Tween 80 in an amount of 31.94%; and Labrafil in an amount of 13.31% (w/w), wherein the % (w/w) values are based on the total weight of the pharmaceutical composition;

(vi) a formulation comprising gemcitabine HCl in an amount of 2.00% (w/w), water in an amount of 18.26% (w/w); 4.0 N NaOH in an amount of 1.74% (w/w); glycerol in an amount of 2.10 % (w/w); PEG 400 in an amount of 30.20% (w/w); Tween 80 in an amount of 32.30% (w/w) and Labrafil in an amount of 13.40% (w/w), wherein the % (w/w) values are based on the total weight of the pharmaceutical composition;

(vii) a formulation comprising gemcitabine HCl in an amount of 2.00% (w/w), water in an amount of 14.40% (w/w); 4.0 N NaOH in an amount of 5.60% (w/w); glycerol in an amount of 2.10 % (w/w); PEG 400 in an amount of 30.20% (w/w); Tween 80 in an amount of 32.30% (w/w) and Labrafil in an amount of 13.40% (w/w), wherein the % (w/w) values are based on the total weight of the pharmaceutical composition; and

(viii) a formulation comprising gemcitabine HCl in an amount of 2.00% (w/w), water in an amount of 14.30% (w/w); 4.0 N NaOH in an amount of 5.70% (w/w); glycerol in an amount of 2.10 % (w/w); PEG 400 in an amount of 30.20% (w/w); Tween 80 in an amount of 32.30% (w/w) and Labrafil in an amount of 13.40% (w/w), wherein the % (w/w) values are based on the total weight of the pharmaceutical composition.

**Patentansprüche**

1. Orale selbst-mikroemulgierende pharmazeutische Zusammensetzung, welche ausgewählt ist aus der Gruppe bestehend aus:

(i) einer Formulierung, die umfasst, Gemcitabin oder dessen pharmazeutisch annehmbares Salz in einer Menge von 2,00% (w/w), Wasser in einer Menge von 20,00% (w/w); Glycerin und PEG zusammen in einer Menge von 32,30% (w/w); und Polysorbat und Oleoylpolyoxylglyceride zusammen in einer Menge von 45,70% (w/w), worin die %- (w/w) Werte auf das Gesamtgewicht der pharmazeutischen Zusammensetzung bezogen sind;

(ii) einer Formulierung, die umfasst, Gemcitabin in einer Menge von 2,00% (w/w); Wasser in einer Menge von 20,00% (w/w); Propylenglykol und PEG in einer Menge von 32,30% (w/w); und Polysorbat und Oleoylpolyoxylglyceride in einer Menge von 45,70% (w/w), worin die %- (w/w) Werte auf das Gesamtgewicht der pharmazeutischen Zusammensetzung bezogen sind; und

(iii) einer Formulierung, die umfasst, Gemcitabin in einer Menge von 1,98% (w/w); Wasser in einer Menge von 19,8% (w/w); Glycerin Und PEG zusammen in einer Menge von 31,98% (w/w); Polysorbat und Oleoylpolyoxylglyceride zusammen in einer Menge von 45,25% (w/w); und D-α-Tocopherylpolyethylenglykol-1000-succinat (TPGS) in einer Menge von 0,99% (w/w), worin die %- (w/w) Werte auf das Gesamtgewicht der pharmazeutischen Zusammensetzung bezogen sind.

2. Orale selbst-mikroemulgierende pharmazeutische Zusammensetzung, welche ausgewählt ist aus der Gruppe bestehend aus:

(i) einer Formulierung, die umfasst, Gemcitabin-HCl in einer Menge von 2,00% (w/w), Wasser in einer Menge von 20,00% (w/w); Glycerin in einer Menge von 2,10 % (w/w); PEG-400 in einer Menge von 30,20% (w/w); Tween-80 in einer Menge von 32,30% (w/w) und Labrafil in einer Menge von 13,40% (w/w), worin die %- (w/w) Werte auf das Gesamtgewicht der pharmazeutischen Zusammensetzung bezogen sind;

(ii) einer Formulierung, die umfasst, Gemcitabin-HCl in einer Menge von 2,00% (w/w); Wasser in einer Menge von 20,00% (w/w); Propylenglykol in einer Menge von 2,10% (w/w) und PEG-400 in einer Menge von 30,20% (w/w); Tween-80 in einer Menge von 32,30% und Labrafil in einer Menge von 13,40% (w/w), worin die %- (w/w) Werte auf das Gesamtgewicht der pharmazeutischen Zusammensetzung bezogen sind;

(iii) einer Formulierung, die umfasst, Gemcitabin-HCl in einer Menge von 1,98% (w/w); Wasser in einer Menge von 19,8% (w/w); Glycerin in einer Menge von 2,08% (w/w); PEG-400 in einer Menge von 29,90% (w/w); D-α-Tocopherylpolyethylenglykol-1000-succinat (TPGS) in einer Menge von 0,99% (w/w); Tween-80 in einer Menge von 31,94%; und Labrafil in einer Menge von 13,31% (w/w), worin die %- (w/w) Werte auf das Gesamtgewicht der pharmazeutischen Zusammensetzung bezogen sind;

(iv) einer Formulierung, die umfasst, Gemcitabin-HCl in einer Menge von 2,00% (w/w), Wasser in einer Menge von 18,03% (w/w); 4,0 N NaOH in einer Menge von 1,97% (w/w); Glycerin in einer Menge von 2,10 % (w/w); PEG-400 in einer Menge von 30,20% (w/w); Tween-80 in einer Menge von 32,30% (w/w) und Labrafil in einer Menge von 13,40% (w/w), worin die %- (w/w) Werte auf das Gesamtgewicht der pharmazeutischen Zusammensetzung bezogen sind;

(v) einer Formulierung, die umfasst, Gemcitabin-HCl in einer Menge von 1,98% (w/w); Wasser in einer Menge von 17,85% (w/w); 4,0 N NaOH in einer Menge von 1,95% (w/w); Propylenglycerin in einer Menge von 2,08% (w/w); PEG-400 in einer Menge von 29,90% (w/w); D-$\alpha$-Tocopherylpolyethylenglykol-1000-succinat (TPGS) in einer Menge von 0,99% (w/w); Tween-80 in einer Menge von 31,94%; und Labrafil in einer Menge von 13,31% (w/w), worin die %- (w/w) Werte auf das Gesamtgewicht der pharmazeutischen Zusammensetzung bezogen sind;

(vi) einer Formulierung, die umfasst, Gemcitabin-HCl in einer Menge von 2,00% (w/w), Wasser in einer Menge von 18,26% (w/w); 4,0 N NaOH in einer Menge von 1,74% (w/w); Glycerin in einer Menge von 2,10 % (w/w); PEG-400 in einer Menge von 30,20% (w/w); Tween-80 in einer Menge von 32,30% (w/w) und Labrafil in einer Menge von 13,40% (w/w), worin die %- (w/w) Werte auf das Gesamtgewicht der pharmazeutischen Zusammensetzung bezogen sind;

(vii) einer Formulierung, die umfasst, Gemcitabin-HCl in einer Menge von 2,00% (w/w), Wasser in einer Menge von 14,40% (w/w); 4,0 N NaOH in einer Menge von 5,60% (w/w); Glycerin in einer Menge von 2,10 % (w/w); PEG-400 in einer Menge von 30,20% (w/w); Tween-80 in einer Menge von 32,30% (w/w) und Labrafil in einer Menge von 13,40% (w/w), worin die %- (w/w) Werte auf das Gesamtgewicht der pharmazeutischen Zusammensetzung bezogen sind; und

(viii) einer Formulierung, die umfasst, Gemcitabin-HCl in einer Menge von 2,00% (w/w), Wasser in einer Menge von 14,30% (w/w); 4,0 N NaOH in einer Menge von 5,70% (w/w); Glycerin in einer Menge von 2,10 % (w/w); PEG-400 in einer Menge von 30,20% (w/w); Tween-80 in einer Menge von 32,30% (w/w) und Labrafil in einer Menge von 13,40% (w/w), worin die %- (w/w) Werte auf das Gesamtgewicht der pharmazeutischen Zusammensetzung bezogen sind.

## Revendications

1. Composition pharmaceutique auto-micro-émulsifiante par voie orale, qui est choisie dans le groupe constitué par :

(i) une formulation comprenant de la gemcitabine ou son sel pharmaceutiquement acceptable en une quantité de 2,00% (P/P) ; de l'eau en une quantité de 20,00% (P/P) ; du glycérol et du PEG ensemble en une quantité de 32,30% (P/P) ; et du polysorbate et des polyoxylglycérides d'oléoyle ensemble en une quantité de 45,70% (P/P), les valeurs de % (P/P) étant basées sur le poids total de la composition pharmaceutique ;

(ii) une formulation comprenant de la gemcitabine en une quantité de 2,00% (P/P) ; de l'eau en une quantité de 20,00% (P/P) ; du propylèneglycol et du PEG en une quantité de 32,30% (P/P) ; et du polysorbate et des polyoxylglycérides d'oléoyle en une quantité de 45,70% (P/P), les valeurs de % (P/P) étant basées sur le poids total de la composition pharmaceutique ; et

(iii) une formulation comprenant de la gemcitabine en une quantité de 1,98% (P/P) ; de l'eau en une quantité de 19,8% (P/P) ; du glycérol et du PEG ensemble en une quantité de 31,98% (P/P) ; du polysorbate et des polyoxylglycérides d'oléoyle ensemble en une quantité de 45,25% (P/P) ; et du D-$\alpha$-tocophéryl polyéthylèneglycol 1000 succinate (TPGS) en une quantité de 0,99% (P/P), les valeurs de % (P/P) étant basées sur le poids total de la composition pharmaceutique.

2. Composition pharmaceutique auto-micro-émulsifiante par voie orale, qui est choisie dans le groupe constitué par :

(i) une formulation comprenant du gemcitabine.HCl en une quantité de 2,00% (P/P) ; de l'eau en une quantité de 20,00% (P/P) ; du glycérol en une quantité de 2,10% (P/P) ; du PEG 400 en une quantité de 30,20% (P/P) ; du Tween 80 en une quantité de 32,30% (P/P) ; et du Labrafil en une quantité de 13,40% (P/P), les valeurs de % (P/P) étant basées sur le poids total de la composition pharmaceutique ;

(ii) une formulation comprenant du gemcitabine.HCl en une quantité de 2,00% (P/P) ; de l'eau en une quantité de 20,00% (P/P) ; du propylèneglycol en une quantité de 2,10% (P/P) et du PEG 400 en une quantité de 30,20% (P/P) ; du Tween 80 en une quantité de 32,30% ; et du Labrafil en une quantité de 13,40% (P/P), les valeurs de % (P/P) étant basées sur le poids total de la composition pharmaceutique ;

(iii) une formulation comprenant du gemcitabine.HCl en une quantité de 1,98% (P/P) ; de l'eau en une quantité de 19,8% (P/P) ; du glycérol en une quantité de 2,08% (P/P) ; du PEG 400 en une quantité de 29,90% (P/P) ; du D-$\alpha$-tocophéryl polyéthylèneglycol 1000 succinate (TPGS) en une quantité de 0,99% (P/P) ; du Tween 80 en une quantité de 31,94% ; et du Labrafil en une quantité de 13,31% (P/P), les valeurs de % (P/P) étant basées sur le poids total de la composition pharmaceutique ;

(iv) une formulation comprenant du gemcitabine.HCl en une quantité de 2,00% (P/P) ; de l'eau en une quantité de 18,03% (P/P) ; du NaOH à 4,0 N en une quantité de 1,97% (P/P) ; du glycérol en une quantité de 2,10% (P/P) ; du PEG 400 en une quantité de 30,20% (P/P) ; du Tween 80 en une quantité de 32,30% (P/P) ; et du Labrafil en une quantité de 13,40% (P/P), les valeurs de % (P/P) étant basées sur le poids total de la composition pharmaceutique ;

(v) une formulation comprenant du gemcitabine.HCl en une quantité de 1,98% (P/P) ; de l'eau en une quantité de 17,85% (P/P) ; du NaOH à 4,0 N en une quantité de 1,95% (P/P) ; du propylèneglycérol en une quantité de 2,08% (P/P) ; du PEG 400 en une quantité de 29,90% (P/P) ; du D-$\alpha$-tocophéryl polyéthylèneglycol 1000 succinate (TPGS) en une quantité de 0,99% (P/P) ; du Tween 80 en une quantité de 31,94% ; et du Labrafil en une quantité de 13,31% (P/P), les valeurs de % (P/P) étant basées sur le poids total de la composition pharmaceutique ;

(vi) une formulation comprenant du gemcitabine.HCl en une quantité de 2,00% (P/P), de l'eau en une quantité de 18,26% (P/P) ; du NaOH à 4,0 N en une quantité de 1,74% (P/P) ; du glycérol en une quantité de 2,10% (P/P) ; du PEG 400 en une quantité de 30,20% (P/P) ; du Tween 80 en une quantité de 32,30% (P/P) ; et du Labrafil en une quantité de 13,40% (P/P), les valeurs de % (P/P) étant basées sur le poids total de la composition pharmaceutique ;

(vii) une formulation comprenant du gemcitabine.HCl en une quantité de 2,00% (P/P) ; de l'eau en une quantité de 14,40% (P/P) ; du NaOH à 4,0 N en une quantité de 5,60% (P/P) ; du glycérol en une quantité de 2,10% (P/P) ; du PEG 400 en une quantité de 30,20% (P/P) ; du Tween 80 en une quantité de 32,30% (P/P) ; et du Labrafil en une quantité de 13,40% (P/P), les valeurs de % (P/P) étant basées sur le poids total de la composition pharmaceutique ; et

(viii) une formulation comprenant du gemcitabine.HCl en une quantité de 2,00% (P/P) ; de l'eau en une quantité de 14,30% (P/P) ; du NaOH à 4,0 N en une quantité de 5,70% (P/P) ; du glycérol en une quantité de 2,10% (P/P) ; du PEG 400 en une quantité de 30,20% (P/P) ; du Tween 80 en une quantité de 32,30% (P/P) ; et du Labrafil en une quantité de 13,40% (P/P), les valeurs de % (P/P) étant basées sur le poids total de la composition pharmaceutique.

Figure 1

Figure 2

**EP 3 045 165 B1**

**REFERENCES CITED IN THE DESCRIPTION**

**Patent documents cited in the description**

- US 7220428 B
- US 7053076 B
- US 7217735 B
- US 7309696 B
- WO 2004017944 A
- WO 2007089043 PCT
- US 2003104048 A
- US 2004115287 A